# FASCICULE DE BREVET EUROPEEN

(11) **EP 2 371 950 B1**
(45) Date de publication et mention de la délivrance du brevet: **11.09.2013**
(21) Numéro de dépôt: 11290113.7
(22) Date de dépôt: 04.03.2011
(51) Int. Cl.: C12N 9/42, C12P 21/00, C12N 1/14, C12N 9/24

(54) **Procédé de production de cellulases basé sur la régulation de l'oscillation de la pression en oxygène dissous dans le milieu de culture**
Verfahren zur Production von Zellulasen basiert an der Regulierung der Druckschwingung des im kulturmedium aufgelössten Sauerstoffs.
Method for the production of cellulases based on the regulation of the oscillation of the pression of the oxygen disolved in the culture medium

(30) Priorité: 26.03.2010 FR 1001211
(43) Date de publication de la demande: 05.10.2011
(73) Titulaire: IFP Energies nouvelles, 92852 Rueil-Malmaison Cedex (FR)
(72) Inventeur: Ben Chaabane, Fadhel, 75020 Paris (FR); Cohen, Céline, 75017 Paris (FR)

(56) Documents cités:
- EP-A1- 1 690 944
- US-A- 4 952 505
- RECZEY K ET AL: "Cellulase production by T. reesei", BIORESOURCE TECHNOLOGY, vol. 57, no. 1, 1996, pages 25-30, XP002598219, ISSN: 0960-8524

## Description

### DOMAINE DE L'INVENTION

L'invention est relative à un procédé de production d'enzymes pour l'hydrolyse de biomasse lignocellulosique.

### ART ANTÉRIEUR

La biomasse lignocellulosique se caractérise par une structure complexe constituée de trois principaux polymères : la cellulose, l'hémicellulose et la lignine. La cellulose et éventuellement les hémicelluloses sont les cibles de l'hydrolyse enzymatique mais ne sont pas directement accessibles aux enzymes.

C'est la raison pour laquelle ces substrats doivent subir un prétraitement précédant l'étape d'hydrolyse enzymatique. Le prétraitement vise à modifier les propriétés physiques et physicochimiques du matériau lignocellulosique, en vue d'améliorer l'accessibilité de la cellulose emprisonnée dans la matrice de lignine et d'hémicellulose. Ces prétraitements peuvent être de différents types : on peut,citer les cuissons acides, les cuissons alcalines, l'explosion à la vapeur, ou encore les procédés Organosolv.

L'étape d'hydrolyse enzymatique permet la transformation de la cellulose et des hémicelluloses en sucres par utilisation d'enzymes cellulolytiques et/ou hémicellulolytiques. Des microorganismes, comme les champignons appartenant aux genres *Trichoderma, Aspergillus, Penicillium ou Schizophyllum,* ou les bactéries anaérobies appartenant par exemple au genre *Clostridium,* produisent ces enzymes, contenant notamment les cellulases et les hémicellulases, adaptées à l'hydrolyse totale de la cellulose et des hémicelluloses.

Les sucres obtenus par hydrolyse de la biomasse lignocellulosique sont des pentoses (xylose et arabinose principalement), des disaccharides (cellobiose) et du glucose qui peuvent être fermentés par les microorganismes. Le glucose peut, par exemple, être facilement transformé en éthanol par la levure *Saccharomyces cerevisiae* lors de l'étape de fermentation alcoolique.

Enfin, une étape de distillation permet de séparer et de récupérer le produit issu de la fermentation ainsi obtenu, donc l'éthanol dans le cas précédent, du moût de fermentation.

Les différentes études technico-économiques démontrent la nécessité de réduire le coût liés à l'étape d'hydrolyse enzymatique pour amener le coût de l'éthanol produit à des valeurs proches de l'éthanol obtenu à partir d'amidon.

Un des moyens de diminution des coûts consiste à optimiser les conditions opératoires du procédé de production de cellulases de façon à augmenter la productivité ou à obtenir un cocktail enzymatique ayant une activité spécifique améliorée.

Le microorganisme le plus utilisé pour la production de cellulases est le champignon filamenteux *Trichoderma reesei.* Les souches sauvages ont la faculté de secréter, en présence d'un substrat inducteur comme par exemple la cellulose ou le lactose, un complexe enzymatique bien adapté à l'hydrolyse de la cellulose. Les enzymes du complexe enzymatique contiennent trois grands types d'activité : les endoglucanases, les exoglucanases et les cellobiases.

Les mécanismes complexes de régulation de la synthèse des cellulases nécessitent des mises en oeuvre particulières pour leur production en réacteur. L'emploi de souches non sensibles à la répression catabolique et de substrats inducteurs solubles et utilisables à grande échelle comme le lactose a permis d'obtenir des productions importantes de l'ordre de 40g/l de protéines extracellulaires par *T.reesei* CL 847 selon un procédé décrit dans Bioresource Technol.(1992) 39, 125-130. Dans ce procédé, la fermentation se déroule en deux phases, une première phase en batch de production de cellules de *T.reesei,* et une seconde phase d'alimentation en fed-batch de l'inducteur à une vitesse évitant son accumulation dans le milieu.

L'état physiologique du microorganisme n'étant pas toujours maintenu à son optimum pour la production d'enzymes, on observe dans certains cas des phénomènes de croissance de cellules non productives, de lyse cellulaire ou de sporulation non souhaitée, entraînant diminution de la productivité en enzymes. L'équilibre entre les états de production optimale et les états physiologiques indésirables est assez sensible. Bailey et al. (Appl. Microbiol. Biotechnol. 2003 62:156-162) ont montré que la productivité des cellulases était optimale lorsque les cellules étaient dans un état proche de la limitation en substrat carboné dans le milieu. Il a proposé un procédé d'ajout de substrat carboné mettant en oeuvre le suivi de la vitesse de consommation de base utilisée pour le contrôle du pH.

Par ailleurs, le procédé décrit dans le brevet EP-B1-0448430 nécessite un apport optimisé du flux de sucre de 35 à 45 mg.g⁻¹.h⁻¹. En effet, un flux trop important aboutit à une accumulation de substrat carboné dans le milieu, ce qui conduit à un changement métabolique vers la production de biomasse à la place de la production d'enzymes. Un flux trop faible conduit quant à lui à une lyse de la biomasse et à une perte de production. De plus, *Trichoderma reesei* excrète alors plus de protéases reconsommant une partie des cellulases produites.

La présente invention propose un procédé permettant de maintenir le microorganisme dans l'état correspondant à son optimum pour la production d'enzymes.

### RÉSUMÉ DE L'INVENTION

L'invention propose un procédé de production d'enzymes cellulotiques et/ou hémicellulolytiques dans lequel l'apport de substrat inducteur carboné nécessaire pour la phase de production est régulé par une oscillation de la pression en oxygène dissous dans le milieu.

### DESCRIPTION DES DESSINS

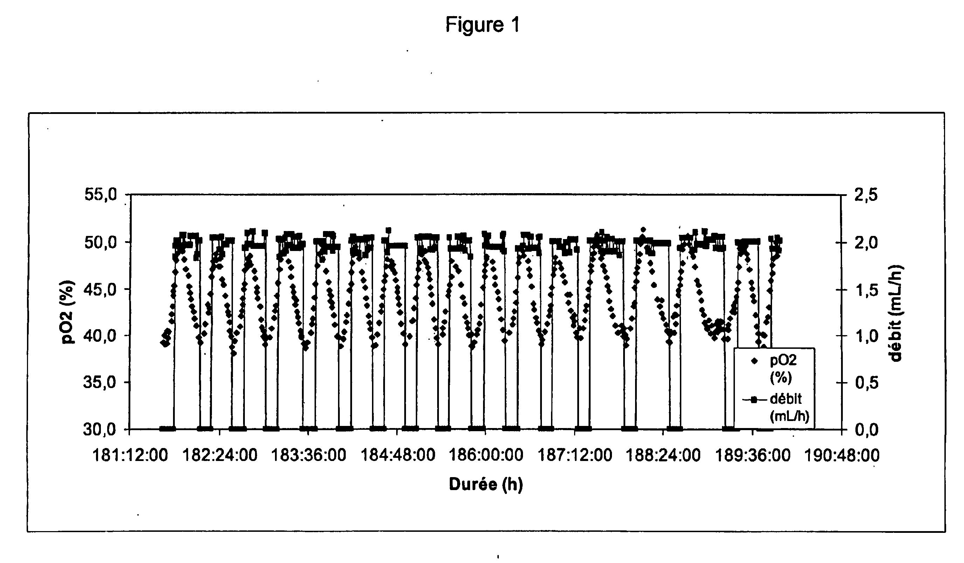

La Figure 1 représente l'enregistrement de la pression en oxygène dissous dans le milieu en fonction du temps ainsi que le débit d'apport de substrat carboné.

### DESCRIPTION DÉTAILLÉE DE L'INVENTION

La présente invention porte sur un procédé de production d'enzymes cellulolytiques et/ou hémicellulolytiques par un microorganisme cellulolytique en bioréacteur agité et aéré comprenant une phase de croissance en présence d'une source de carbone et une phase de production en présence d'un substrat inducteur carboné dans lequel l'apport de substrat inducteur carboné pendant la phase de production est régulé en fonction de la pression partielle en oxygène dissous dans le milieu de croissance, ladite pression oscillant entre deux valeurs Po₂ₘᵢₙ et Po₂ₘₐₓ, la valeur de Po₂ₘᵢₙ étant supérieure à la pression critique Po_{2critique} en dessous de laquelle l'activité du microorganisme est affectée et inférieure à 95% de la pression partielle en oxygène à saturation Po₂ₛₐₜ fixée lors du démarrage du bioréacteur et, la valeur de Po₂ₘₐₓ étant supérieure d'au moins 5% à Po₂ₘᵢₙ,
l'ajout de substrat inducteur carboné étant effectué dès que la pression partielle en oxygène dissous dans le milieu est supérieure à la valeur Po₂ₘₐₓ et est stoppé dès que la pression en oxygène dissous dans le milieu est inférieure à la valeur Po₂ₘᵢₙ.

Ce procédé permet d'optimiser la production d'enzymes cellulolytiques et/ou hémicellulolytiques en maintenant les cellules dans une phase de productivité optimale.

Grâce au procédé selon l'invention, il est possible de produire un cocktail enzymatique présentant une activité spécifique jusqu'à 50% plus importante que celle d'enzymes obtenues avec un procédé classique de production où la limitation en carbone est imposée avec un débit constant.

De plus, ce procédé présente l'avantage d'être robuste et simple à mettre en oeuvre. Il permet également d'éviter l'accumulation de sucres dans le milieu, qui conduit à la formation de biomasse au dépend de la production d'enzymes.

Les souches de microorganismes utilisées sont cultivées en bioréacteurs agités et aérés dans des conditions compatibles avec leur croissance et la production d'enzymes.

Au démarrage de la fermentation, le bioréacteur est saturé en oxygène et la pression partielle en oxygène dissous dans le milieu correspondant à la saturation est notée Po₂ₛₐₜ.

La source de carbone utilisée pour la phase de croissance est introduite dans le bioréacteur de façon à avoir une concentration initiale en sucre pour le démarrage de la phase de production comprise entre 15 et 60 g/L.

Durant la phase de croissance, l'aération est fixée à une valeur choisie par l'opérateur et la pression partielle en oxygène Po₂ est régulée à un pourcentage, défini par l'opérateur, de la pression à saturation grâce à l'agitation. La pression partielle en oxygène dans le milieu doit être supérieure à la pression critique en oxygène Po_{2critique} en dessous de laquelle l'activité du microorganisme est affectée.

Cette valeur de Po_{2critique} est connue de l'homme du métier et est dépendante du microorganisme utilisé. Elle peut se définir comme étant la pression en dessous de laquelle le métabolisme du microorganisme est affecté par une baisse de la viabilité cellulaire ou de la production de métabolites d'intérêts.

Durant la phase de production, l'agitation et l'aération dans le bioréacteur sont fixées à une valeur prédéfinie permettant d'avoir une capacité d'oxygénation dudit bioréacteur, correspondant à un Kla compris entre 50 et 150 h⁻¹ selon la biomasse visée initialement.

Dans le procédé selon l'invention, le substrat inducteur carboné est introduit après épuisement du substrat initial : il s'agit du démarrage de la phase de production.

La vitesse de consommation d'oxygène dans le milieu est proportionnelle à la vitesse de consommation de substrat inducteur carboné par le microorganisme.

Ainsi, une faible accumulation de sucre dans le fermenteur conduit à une chute de la pression partielle en oxygène. Lorsque la pression partielle en oxygène mesurée atteint une valeur minimale Po_{2min,} la régulation du système stoppe l'ajout de substrat inducteur carboné. Cet arrêt provoque une diminution de la vitesse de consommation du substrat inducteur carboné au moment où la concentration de celui-ci est proche de la constante d'affinité Ks du microorganisme pour le substrat carboné. L'arrêt provoque également une diminution de la vitesse de consommation d'oxygène, ce qui se traduit par une augmentation de la pression partielle en oxygène jusqu'à une valeur Po₂ₘₐₓ. Lorsque la pression Po₂ₘₐₓ est atteinte, la régulation du système fait redémarrer la pompe d'ajout du substrat inducteur carboné.

Les valeurs Po₂ₘᵢₙ et Po₂ₘₐₓ entre lesquelles la pression partielle en oxygène dissous dans le milieu de fermentation oscille sont déterminées par l'opérateur à partir de la pression de saturation en oxygène Po₂ₛₐₜ, en fonction des souches de microorganismes utilisées et/ou du milieu de fermentation.

L'amplitude des oscillations entre les valeurs Po₂ₘᵢₙ et Po₂ₘₐₓ régule l'intervalle de temps entre les moments où le substrat carboné inducteur est injecté et les moments où l'injection est stoppée.

Ainsi, les cellules sont par conséquent maintenues dans un état proche de la limitation en carbone (à une concentration proche du Ks), donc favorable à une production optimale d'enzymes cellulolytiques et/ou hémicellulolytiques.

Selon le procédé de la présente invention, l'ajout de substrat inducteur carboné est effectué dès que la pression partielle en oxygène dissous dans le milieu est supérieure à la valeur Po₂ₘₐₓ et est stoppé dès que la pression en oxygène dissous dans le milieu est inférieure à Po₂ₘᵢₙ. Les valeurs Po₂ₘₐₓ et Po₂ₘᵢₙ sont déterminées en fonction de la pression critique en oxygène et de la pression de saturation du milieu en oxygène Po₂ₛₐₜ lors du démarrage du bioréacteur.

La valeur Po₂ₘᵢₙ doit être supérieure à la pression critique Po_{2critique} en dessous de laquelle le métabolisme du microorganisme est affecté et est inférieure à 95% de la pression partielle en oxygène à saturation Po₂ₛₐₜ fixée lors du démarrage du bioréacteur.

De façon préférée, Po₂ₘᵢₙ est comprise entre 10 et 60% de Po₂ₛₐₜ.

La valeur Po₂ₘₐₓ doit être supérieure d'au moins 5% de la valeur de Po₂ₘᵢₙ. Elle peut représenter 100% de la pression partielle en oxygène Po₂ₛₐₜ.

Préférentiellement Po₂ₘₐₓ est comprise entre 30 et 70% de Po₂ₛₐₜ.

Selon un mode très préféré, Po₂ₘₐₓ est comprise entre 40 et 60% de Po₂ₛₐₜ et Po₂ₘᵢₙ est comprise entre 30 et 50% de Po₂ₛₐₜ.

Le substrat inducteur carboné est choisi parmi le lactose, le xylose, le cellobiose, le sophorose, les résidus obtenus après fermentation éthanolique des sucres monomères des hydrolysats enzymatiques de biomasse cellulosique et/ou un extrait brut de pentoses hydrosolubles provenant du prétraitement d'une biomasse cellulosique.

Selon un mode de réalisation préféré, l'apport de substrat est effectué en solution.

Une solution aqueuse contenant le substrat inducteur carboné choisi pour la phase de production des enzymes est préparée à la concentration de 10 à 800 g/L et de façon préférée entre 150 et 600g/L.

La solution aqueuse comprenant le substrat inducteur carboné est de préférence une solution de lactose.

Avantageusement, on utilisera une solution de lactose à 250g/L.

Le substrat carboné utilisé durant la phase batch est choisi parmi le glucose, le galactose, le glycérol, les résidus obtenus après fermentation éthanolique des sucres monomères des hydrolysats enzymatiques de biomasse cellulosique et/ou un extrait brut de pentoses hydrosolubles provenant du prétraitement d'une biomasse cellulosique.

Le microorganisme cellulolytique est choisi parmi les champignons ou d'autres microorganismes modifiés (levures, bactéries). De façon préférée, le microorganisme cellulolytique appartient aux genres *Trichoderma, Aspergillus, Penicillium* et *Schizophyllum,* et de façon très préférée à l'espèce *Trichoderma reesei.*

Le procédé décrit dans la présente invention propose une régulation de l'apport de substrat inducteur carboné en fonction de la pression partielle en oxygène dissous dans le milieu. Une régulation en fonction de la vitesse de production de dioxyde de carbone ou de consommation de dioxygène calculées à partir de la composition du gaz mesurée en sortie constituerait un moyen équivalent de réaliser la présente invention.

### EXEMPLES

Parmi les exemples qui suivent, l'exemple 1 présente la fermentation de référence utilisant le lactose comme substrat carboné de production avec un débit optimisé de 35 à 45 mg.g⁻¹.h⁻¹ qui permet une forte production de protéines.

L'exemple 2 représente la même expérience avec un flux de substrat carboné doublé durant la phase de production. Cet exemple a conduit à une forte accumulation de biomasse. Il démontre le risque encouru lorsque le flux de substrat carboné est supérieur au flux optimal.

Les exemples 3 à 5 sont ceux conformes au procédé selon la présente invention. L'exemple 3 reprend le même débit que l'exemple 1 et l'exemple 4 le même débit que l'exemple 2. Ce dernier exemple démontre la robustesse du procédé puisqu'il a abouti à une forte production de protéines et qu'il n'y a pas eu d'accumulation de biomasse durant la phase de production. L'exemple 5 illustre une plage d'oscillations plus large.

### Exemple 1 (comparatif) : Production d'enzymes selon un procédé de fermentation classique tel que décrit dans le brevet FR-B-2 811 753.

La production de cellulases est effectuée en fermenteur agité mécaniquement. Le milieu minéral a la composition suivante : KOH 1,66 g/L, H₃PO₄ 85 % 2 mUL, (NH₄)₂SO₄ 2,8 g/L, MgSO₄ 7 H₂O 0,6 g/L, CaCL2 0,6 g/L, MnSO₄ 3,2 mg/L, ZnSO₄, 7 H₂O 2,8 mg/L, CoCl₂ 10 4,0 mg/L, FeSO₄, 7 H₂O 10 mg/L, Corn Steep 1,2 g/L, antimousse 0,5 mUL.

Le fermenteur contenant le milieu minéral est stérilisé à 120 °C pendant 20 minutes, la source carbonée glucose est stérilisée à part à 120°C pendant 20 minutes puis ajoutée stérilement dans le fermenteur de façon à avoir une concentration finale de 15 g/L. Le fermenteur est ensemencé à 10% (v/v) avec une préculture liquide de la souche de *Trichoderma reesei* CL847. Le milieu minéral de la préculture, est identique à celui du fermenteur mis à part l'addition de phtalate de potassium à 5 g/L pour tamponner le pH. La croissance du champignon en préculture est faite en utilisant le glucose comme substrat carboné, à la concentration de 30 g/L. La croissance de l'inoculum dure de 2 à 3 jours est effectuée à 28 °C dans un incubateur agité. Le transfert vers le fermenteur est réalisé si la concentration résiduelle en glucose est inférieure à 15 g/L.

L'expérience effectuée en bioréacteur comporte deux phases :
- une phase de croissance sur substrat carboné glucose (concentration initiale 15 g/L) à une température de 27 °C et un pH de 4,8 (régulé par de l'ammoniaque 5,5 M). L'aération est de 0,5 vvm (volume/volume/minute) et la pression partielle en oxygène dissous dans le milieu est de 40 % de PO₂ₛₐₜ.
- une phase de production d'enzymes. Lorsque le substrat initial du fermenteur est épuisé, la solution de lactose à 250 g/L est injectée en continu à 2mL/h correspondant au débit de 35 à 45 mg par g de cellules et par heure jusqu'à 200 heures. La température est baissée à 25 °C et le pH est maintenu à 4 jusqu'à la fin de la culture. Le pH est régulé par addition d'une solution d'ammoniaque à 5,5 N qui apporte l'azote nécessaire à la synthèse des protéines sécrétées. La teneur en oxygène dissous est maintenue à 40 % de PO₂ₛₐₜ.

La production d'enzymes est suivie par le dosage des protéines extracellulaires par la méthode de Lowry et standard BSA, après séparation du mycélium par filtration ou centrifugation. Les activités cellulolytiques déterminées sont:
- l'activité papier filtre (UPF : unité papier filtre) qui permet de doser l'activité globale du pool enzymatique endoglucanases et exoglucanases
- l'activité aryl β-glucosidase.

L'activité UPF est mesurée sur papier Whatman n° 1 (Procédure recommandée par la commission biotechnologique IUPAC) à la concentration initiale de 50 g/L ; on détermine la prise d'essai de la solution enzymatique à analyser qui libère l'équivalent de 2 g/L de glucose (dosage colorimétrique) en 60 minutes. Le principe de l'activité papier filtre est de déterminer par dosage au DNS (acide dinitrosalicylique) la quantité de sucres réduits issue d'un papier Whatman N°1.

Le substrat utilisé pour déterminer l'activité aryl β-glucosidase est le p-nitrophenyl-β-D-glucopyranoside (PNPG). Il est clivé par la β-glucosidase qui libère le p-nitrophenol.

Une unité d'activité aryl β-glucosidase est définie comme la quantité d'enzyme nécessaire pour produire 1 µmol de p-nitrophenol à partir de PNPG par minute et est exprimé en IU/mL.

Les activités spécifiques sont obtenues en divisant les activités exprimées en IU/mL par la concentration en protéines. Elles sont exprimées en IU/mg.

Les déterminations analytiques sur le moût final donnent les résultats suivants :
Biomasse g/L 14,5
Protéines g/L 35,6
UPF IU/mL 18,7
UPF spécifique IU/mg 0,52
aryl β-Glucosidase spécifique 0,95 IU/mg

### Exemple 2 (comparatif) : Production d'enzymes avec un flux de substrat carboné durant la phase de fed-batch doublé par rapport à celui de l'exemple 1.

La production d'enzymes est menée dans les mêmes conditions que dans l'Exemple 1. Après 30 heures de croissance, après l'épuisement du substrat initial, la solution de fed-batch est injectée avec un débit doublé (4mL/h au lieu de 2mL/h) par rapport à l'expérience de l'exemple 1. Cela aboutit à une formation importante de biomasse et une faible production de protéines. Le rendement de production de protéines par rapport au substrat carboné consommé est de 0,1 g/g alors qu'il est de 0,3 g/g dans l'exemple 1.

Les déterminations analytiques sur le moût final donnent les résultats suivants :
Biomasse g/L 35,4
Protéines g/L 16,8
UPF IU/mL 11,7
UPF spécifique 0,70 IU/mg
aryl β-Glucosidase spécifique 2,02 IU/mg

### Exemple 3 (selon l'invention) : Production d'enzymes en régulant l'apport du sucre par la régulation de la pression partielle en oxygène dissous dans le milieu (débit 2mL/h

La production d'enzymes est menée dans les mêmes conditions que dans l'Exemple 1. Après l'épuisement du substrat carboné de la phase de croissance. Le débit d'aération est maintenu à 0,5 vvm et l'agitation à 800 rpm. Ce qui permet dans le cas du fermenteur utilisé d'avoir un Kla supérieur à 70 h⁻¹. Le substrat carboné est apporté en fonction de la valeur de la Po₂. Le débit de substrat est déclenché lorsque la pression partielle en oxygène est supérieure à 50% de la pression en oxygène à saturation avec un débit de 2 mL/h et s'arrête lorsque celle-ci est inférieure à 40% de la pression partielle en oxygène à saturation (voir figure 1 ).

Les déterminations analytiques sur le moût final donnent les résultats suivants :
Biomasse g/L 13,5
Protéines g/L 40,6
UPF IU/mL 28,1
UPF spécifique 0,69 IU/mg
aryl β-Glucosidase spécifique 1,97 IU/mg

Le rendement final de production de protéines par rapport au substrat carboné est de 0,4 g/g. L'activité spécifique FPase du cocktail enzymatique produit est améliorée de 33%.

### Exemple 4 : Production d'enzymes en régulant l'apport du sucre par la régulation de la pression partielle en oxygène avec un débit doublé par rapport à l'exemple 3 (débit 4mL/h)

La production d'enzymes est menée dans les mêmes conditions que dans l'Exemple 3. Le substrat carboné est apporté en fonction de la valeur de la Po₂. Le débit de substrat est déclenché lorsque la pression partielle en oxygène est supérieure à 50% de la pression en oxygène à saturation avec un débit de 4 mUh au lieu de 2mUh et s'arrête lorsque celle-ci est inférieure à 40% de la pression en oxygène à saturation. Cela permet de tester l'effet d'un apport plus important en sucre.

Cet exemple démontre la robustesse du procédé. Le fait de doubler le débit n'aboutit pas à une accumulation de la biomasse comme dans le cas de l'exemple 2 grâce à la régulation. Cela signifie que même si la concentration du substrat carboné dans le bac d'alimentation est variable (ce qui peut arriver à l'échelle industrielle), le procédé s'auto-régule pour maintenir le flux optimal pour la production d'enzymes.

Les déterminations analytiques sur le moût final donnent les résultats suivants :
Biomasse g/L 16,1
Protéines g/L 43,6
UPF IU/mL 33,9
UPF spécifique 0,78 IU/mg
aryl β-Glucosidase spécifique 1,60 IU/mg

Le rendement final de production de protéines par rapport au substrat carboné est de 0,4 g/g. L'activité spécifique FPase du cocktail enzymatique produit est améliorée de 50% par rapport à l'exemple 1.

### Exemple 5 : Production d'enzymes en régulant l'apport du sucre par une oscillation de la pression partielle en oxygène entre 20 et 80%

La production d'enzymes est menée dans les mêmes conditions que dans l'Exemple 4. Le substrat carboné est apporté en fonction de la valeur de la Po₂. Le débit de substrat est déclenché lorsque la pression partielle en oxygène est supérieure à 80% de la pression en oxygène à saturation avec un débit de 4 mL/h et s'arrête lorsque celle-ci est inférieure à 20% de la pression en oxygène à saturation. Cela permet de tester l'effet d'une plage d'oscillation plus importante.

Les déterminations analytiques sur le moût final donnent les résultats suivants :
Biomasse g/L 13,6
Protéines g/L 21,8
UPF IU/mL 20,4
UPF spécifique 0,93 IU/mg
β-Glucosidase spécifique 2,14 IU/mg

La production finale en protéines est plus faible car la quantité de substrat injectée est plus faible. On constate toutefois que les activités spécifiques du cocktail enzymatique sont plus élevées que ceux de l'exemple 4 (oscillation de la Po₂ entre 40 et 50%). Les conditions utilisées au cours de l'exemple 4 sont plus intéressantes car elles permettent d'avoir une productivité et un rendement plus important.

## Revendications

1. Procédé de production d'enzymes cellulolytiques et/ou hémicellulolytiques par un microorganisme cellulolytique en bioréacteur aéré et agité comprenant une phase de croissance en présence d'une source de carbone et une phase de production en présence d'un substrat inducteur carboné dans lequel l'apport de substrat inducteur carboné pendant la phase de production est régulé en fonction de la pression partielle en oxygène dissous dans le milieu de croissance, ladite pression oscillant entre deux valeurs PO₂ₘᵢₙ et PO_{2max,}
la valeur de PO₂ₘᵢₙ étant supérieure à la pression critique PO_{2critique} en dessous de laquelle le métabolisme du microorganisme est affecté et inférieure à 95% de la pression partielle en oxygène à saturation PO₂ₛₐₜ fixée lors du démarrage du bioréacteur et,
la valeur de PO₂ₘₐₓ étant supérieure d'au moins 5% à PO₂ₘᵢₙ.
l'ajout de substrat inducteur carboné étant effectué dès que la pression partielle en oxygène dissous dans le milieu est supérieure à la valeur PO₂ₘₐₓ et est stoppé dès que la pression en oxygène dissous dans le milieu est inférieure à la valeur PO₂ₘᵢₙ.

2. Procédé selon la revendication 1 dans lequel PO₂ₘₐₓ est comprise entre 30 et 70% de PO₂ₛₐₜ et PO₂ₘᵢₙ est comprise 10 et 60% de PO₂ₛₐₜ.

3. Procédé selon la revendication 2 dans lequel PO₂ₘₐₓ est comprise entre 40 et 60% de PO₂ₛₐₜ et PO₂ₘᵢₙ est comprise entre 30 et 50% de PO₂ₛₐₜ.

4. Procédé selon l'une des revendications précédentes dans lequel le substrat inducteur carboné est choisi parmi le lactose, le xylose, le cellobiose, le sophorose, les résidus obtenus après fermentation éthanolique des sucres monomères des hydrolysats enzymatiques de biomasse cellulosique, et/ou un extrait brut de pentoses hydrosolubles provenant du prétraitement d'une biomasse cellulosique.

5. Procédé selon l'une des revendications précédentes dans lequel l'apport de substrat est effectué en solution.

6. Procédé selon la revendication 5 dans lequel le substrat inducteur carboné choisi pour la phase de production des enzymes est en concentration de 10 à 800 g/L, de préférence à la concentration de 150 à 600 g/L .

7. Procédé selon l'une des revendications 5 ou 6 dans lequel le substrat inducteur carboné est une solution de lactose, de préférence à une concentration de 250g/L.

8. Procédé selon l'une des revendications précédentes dans lequel l'agitation et l'aération du bioréacteur sont fixées à une valeur prédéfinie permettant d'avoir une capacité d'oxygénation dudit bioréacteur comprise entre 50 et 150 h⁻¹.

9. Procédé selon l'une des revendications précédentes dans lequel le microorganisme cellulolytique est choisi parmi les champignons appartenant aux genres *Trichoderma, Aspergillus, Penicillium* et *Schizophyllum.*

10. Procédé selon la revendication 9 dans lequel le microorganisme cellulolytique appartient à l'espèce *Trichoderma reesei.*

## Patentansprüche

1. Verfahren zur Herstellung von zellulosespaltenden und/oder hemizellulosespaltenden Enzymen durch einen zellulosespaltenden Mikroorganismus im belüfteten und gerührten Bioreaktor, umfassend eine Wachstumsphase in Gegenwart einer Kohlenstoffquelle und eine Produktionsphase in Gegenwart eines induzierenden kohlenstoffhaltigen Substrats, wobei die Zufuhr des induzierenden kohlenstoffhaltigen Substrats während der Produktionsphase in Abhängigkeit vom im Wachstumsmedium gelösten Sauerstoff-Partialdruck reguliert wird, wobei der Druck zwischen zwei Werten PO₂ₘᵢₙ und PO₂ₘₐₓ schwankt,
wobei der Wert von PO₂ₘᵢₙ größer ist als der kritische Druck PO_{2kritisch}, unterhalb dem der Stoffwechsel des Mikroorganismus beeinflusst wird und unterhalb von 95 % des gesättigten Sauerstoff-Partialdrucks PO₂ₛₐₜ, der beim Starten des Bioreaktors festgelegt wird, und
wobei der Wert von PO₂ₘₐₓ mindestens um 5 % höher ist als PO₂ₘᵢₙ,
wobei die Zugabe des induzierenden kohlenstoffhaltigen Substrats durchgeführt wird, sobald der in dem Medium gelöste SauerstoffPartialdruck größer ist als der Wert PO₂ₘₐₓ und gestoppt wird, sobald der in dem Medium gelöste Sauerstoffdruck unter dem Wert PO₂ₘᵢₙ liegt.

2. Verfahren nach Anspruch 1, wobei PO₂ₘₐₓ im Bereich zwischen 30 und 70 % von PO₂ₛₐₜ und PO₂ₘᵢₙ im Bereich zwischen 10 und 60 % von PO₂ₛₐₜ liegt.

3. Verfahren nach Anspruch 2, wobei PO₂ₘₐₓ im Bereich zwischen 40 und 60 % von PO₂ₛₐₜ und PO₂ₘᵢₙ im Bereich zwischen 30 und 50 % von PO₂ₛₐₜ liegt.

4. Verfahren nach einem der vorhergehenden Ansprüche, wobei das kohlenstoffhaltige induzierende Substrat ausgewählt ist aus Lactose, Xylose, Cellobiose, Sophorose, Resten, die nach ethanolischer Fermentation von monomeren Zuckern enzymatischer Hydrolysate aus zellulosehaltiger Biomasse erhalten wurden, und/oder einem Rohextrakt wasserlöslicher Pentosen, der aus der Vorbehandlung einer zellulosehaltigen Biomasse stammt.

5. Verfahren nach einem der vorhergehenden Ansprüche, wobei die Zufuhr von Substrat in Lösung durchgeführt wird.

6. Verfahren nach Anspruch 5, wobei das kohlenstoffhaltige induzierende Substrat, das für die Produktionsphase der Enzyme ausgewählt wird, in einer Konzentration von 10 bis 800 g/L, bevorzugt in der Konzentration von 150 bis 600 g/L vorliegt.

7. Verfahren nach einem der Ansprüche 5 oder 6, wobei das kohlenstoffhaltige induzierende Substrat eine Lactoselösung, bevorzugt in einer Konzentration von 250 g/L, ist.

8. Verfahren nach einem der vorhergehenden Ansprüche, wobei das Rühren und die Belüftung des Bioreaktors auf einen vordefinierten Wert festgelegt sind, der es ermöglicht, eine Sauerstoffaufnahmekapazität des Bioreaktors im Bereich zwischen 50 und 150 h⁻¹ zu erhalten.

9. Verfahren nach einem der vorhergehenden Ansprüche, wobei der zellulosespaltende Mikroorganismus ausgewählt ist aus den Pilzen, die zu den Gattungen *Trichoderma, Aspergillus, Penicillium* und *Schizophyllum* gehören.

10. Verfahren nach Anspruch 9, wobei der zellulosespaltende Mikroorganismus zur Spezies *Trichoderma reesei* gehört.

## Claims

1. A method of producing cellulolytic and/or hemicellulolytic enzymes by a cellulolytic microorganism in a stirred and aerated bioreactor, comprising a growth stage in the presence of a carbon source and a production stage in the presence of a carbon-containing inductive substrate, wherein the supply of carbon-containing inductive substrate during the production stage is regulated according to the dissolved oxygen partial pressure in the growth medium, said pressure oscillating between two values PO₂ₘᵢₙ and PO₂ₘₐₓ, the value of PO₂ₘᵢₙ being greater than the critical pressure PO_{2critical} below which the activity of the microorganism is affected and less than 95 % of the saturation oxygen partial pressure PO₂ₛₐₜ set when starting the bioreactor, and the value of PO₂ₘₐₓ being at least 5 % greater than PO₂ₘᵢₙ, addition of the carbon-containing inductive substrate being started as soon as the dissolved oxygen partial pressure in the medium is greater than value PO₂ₘₐₓ and stopped as soon as the dissolved oxygen pressure in the medium is below value PO₂ₘᵢₙ.

2. A method as claimed in claim 1, wherein PO₂ₘₐₓ ranges between 30 and 70 % of PO₂ₛₐₜ and PO₂ₘᵢₙ ranges between 10 and 60 % of PO₂ₛₐₜ.

3. A method as claimed in claim 2, wherein PO₂ₘₐₓ ranges between 40 and 60 % of PO₂ₛₐₜ and PO₂ₘᵢₙ ranges between 30 and 50 % of PO₂ₛₐₜ.

4. A method as claimed in any one of the previous claims, wherein the carbon-containing inductive substrate is selected from among lactose, xylose, cellobiose, sophorose, residues obtained after ethanolic fermentation of the monomeric sugars of the enzymatic hydrolysates of cellulosic biomass and/or a crude extract of hydrosoluble pentoses from the pretreatment of a cellulosic biomass.

5. A method as claimed in any one of the previous claims, wherein the substrate is supplied in solution.

6. A method as claimed in claim 5, wherein the carbon-containing inductive substrate selected for the enzyme production stage is at a concentration of 10 to 800 g/L, preferably at a concentration of 150 to 600 g/L.

7. A method as claimed in any one of claims 5 or 6, wherein the carbon-containing inductive substrate is a lactose solution, preferably at a concentration of 250 g/L.

8. A method as claimed in any one of the previous claims, wherein stirring and aeration of the bioreactor are set at a predetermined value allowing to have an oxygenation capacity for said bioreactor ranging between 50 and 150 h⁻¹.

9. A method as claimed in any one of the previous claims, wherein the cellulolytic microorganism is selected from among fungi belonging to the *Trichoderma, Aspergillus, Penicillium* and *Schizophyllum* genera.

10. A method as claimed in claim 9, wherein the cellulolytic microorganism belongs to the *Trichoderma reesei* species.
